# EUROPÄISCHE PATENTSCHRIFT

(11) **EP 2 226 092 B1**
(45) Veröffentlichungstag und Bekanntmachung des Hinweises auf die Patenterteilung: **15.06.2016**
(21) Anmeldenummer: 09154205.0
(22) Anmeldetag: 03.03.2009
(51) Int. Cl.: A45D 34/04, A61B 5/00

(54) **System zur messtechnischen Erfassung der Veränderung von Parametern der Haut**
System for measuring changes in skin parameters
Système de mesure de la modification de paramètres de la peau

(43) Veröffentlichungstag der Anmeldung: 08.09.2010
(73) Patentinhaber: Courage + Khazaka electronic GmbH, 50829 Köln (DE)
(72) Erfinder: Khazaka, Gabriel, 50859, Köln (DE)
(74) Vertreter: dompatent von Kreisler Selting Werner - Partnerschaft von Patent- und Rechtsanwälten mbB

(56) Entgegenhaltungen:
- WO-A-2007/008800
- DE-A1- 10 015 928
- US-A- 4 819 657
- US-A1- 2006 244 961

## Beschreibung

Die Erfindung betrifft eine System zur messtechnischen Erfassung der Veränderung von Parametern der Haut von Lebewesen auf einer zu untersuchenden Hautoberfläche in Abhängigkeit von der Einwirkung einer Substanz nach dem Oberbegriff des Anspruchs 1.

Aus der Europäischen Offenlegungsschrift EP-A-1 166 717 ist eine Vorrichtung zur messtechnischen Erfassung von Parametern der Haut von Lebewesen auf einer zu untersuchenden Hautoberfläche bekannt, bei der die elastischen Eigenschaften einer Oberflächenstruktur mit einer in einem Gehäuse angeordneten Sonde, mit einem Sender und mindestens einem Empfänger, gemessen wird.

Die US 4, 819,657 offenbart eine Allergietestvorrichtung gemäß der Präambel von Anspruch 1. Die Vorrichtung weist ein poröses Element auf, das mit einem Allergen imprägniert ist. Ferner weist die Vorrichtung eine Ladungsplatte und eine Elektrode auf, zwischen denen eine Spannung angelegt wird.

Die bisher bekannten Systeme zur messtechnischen Erfassung von Parametern der Haut von Lebewesen auf einer zu untersuchenden Hautoberfläche werden häufig für Untersuchungen in wissenschaftlichen Studien verwendet, in denen die Wirksamkeit von pharmazeutischen oder kosmetischen Zubereitungen, insbesondere Hautcremes untersucht wird. In solchen Studien wird die Veränderung der Haut in Abhängigkeit von der Einwirkzeit der Substanz dokumentiert. Dafür werden die Hautparametern der Probanden nach bestimmten Einwirkzeiten der Hautcreme erfasst. Bei den bisherigen Studien müssen die Probanden für die Hautmessungen in ein sogenanntes "Probanden-Zentrum" kommen.

Dadurch besteht jedoch der Nachteil, dass nur Probanden untersucht werden können die im Umkreis des "Probanden-Zentrums" wohnen. Auch sind die Untersuchungen für die Probanden sehr zeitaufwändig, so dass beispielsweise Menschen, die berufstätig sind, nicht als Probanden in Frage kommen.

Der Erfindung liegt demzufolge die Aufgabe zugrunde, ein System der eingangs beschriebenen Art zu schaffen, bei dem die Veränderung von Parametern der Haut von Lebewesen in Abhängigkeit von der Einwirkzeit einer auf die zu untersuchende Hautoberfläche auftragbaren Substanz auf einfache Art und Weise festgestellt werden kann.

Ferner liegt der Erfindung die Aufgabe zugrunde ein Verfahren der eingangs beschriebenen Art zu schaffen, bei dem die Veränderung von Parametern der Haut von Lebewesen in Abhängigkeit von der Einwirkzeit einer auf die zu untersuchende Hautoberfläche auftragbaren kosmetischen Substanz auf einfache Art und Weise festgestellt werden kann.

Zur Lösung dieser Aufgabe dienen die Merkmale des Anspruchs 1.

Die Erfindung sieht in vorteilhafter Weise vor, dass bei einem System zur messtechnischen Erfassung der Veränderung von Parametern der Haut von Lebewesen auf einer zu untersuchenden Hautoberfläche in Abhängigkeit von der Einwirkzeit einer Substanz die Messvorrichtung mit mindestens einer Spendeeinrichtung über ein lokales drahtloses Netzwerk gekoppelt ist, wobei die Spendeeinrichtung eingerichtet ist, durch Betätigung eine dieser Spendeeinrichtung zugeordneten und auf die zu untersuchende Hautoberfläche auftragbaren Substanz auszugeben, wobei die Messvorrichtung eingerichtet ist, den Zeitpunkt der Betätigung der Spendeeinrichtung und den Zeitpunkt der mindestens einen Messung der Hautparameter zur Erfassung der Einwirkzeit der Substanz sowie die gemessenen Hautparameter, die dem Zeitpunkt der Messung zugeordnet sind, in einem Speicher auf zu zeichnen.

Dies hat den Vorteil, dass die Messungen in Abhängigkeit der Einwirkzeit gespeichert werden. Dadurch kann die Veränderung der Haut auf einfache Art und Weise dokumentiert werden. Ein solches Gerät können die Probanden mit nach Hause nehmen. Auf diese Weise können auch Probanden an einer Studie teilnehmen, die nicht im näheren Umkreis eines "Probanden-Zentrums" wohnen oder die berufstätig sind.

Ein weiterer Vorteil ist, dass die Hautuntersuchungen auch nach kürzeren Zeitintervallen vorgenommen werden können.

Die Substanz kann eine pharmazeutische oder kosmetische Zubereitung, insbesondere eine Hautcreme sein.

Die Messvorrichtung kann nach einem einstellbaren Zeitintervall oder mehreren einstellbaren Zeitintervallen jeweils ein Erinnerungssignal ausgeben. Dadurch kann den Probanden ein bestimmter Zeitplan vorgegeben werden.

Die Messvorrichtung kann die in der Spendeeinrichtung befindliche Substanz anhand einer der Substanz zugeordneten Identifikationscodierung identifizieren und die zu speichernden Messwerte und Zeitpunkte der Messungen und Zeitpunkte der Betätigungen der Spendeeinrichtung dieser Substanz zuordnen.

Die Messvorrichtung kann die einer bestimmten Substanz zugeordneten Messergebnisse und Zeitpunkte der Messungen und Zeitpunkte der Betätigungen der Spendeeinrichtung an mindestens einen externen Datenempfänger senden oder auf eine auswechselbares Speichermedium übertragen. Auf diese Weise können die Messergebnisse von Probanden, die nicht im näheren Umkreis eines "Probanden-Zentrums" wohnen, auf einfache Art und Weise an das "Probanden-Zentrum" gesendet werden

Die Messvorrichtung kann eine Raumklima-Sensoreinrichtung aufweisen.

Die Messvorrichtung kann die Hautelastizität und/oder die Hautfarbe (Pigmentierung der Haut) und/oder den Fettanteil der Haut und/oder den Talganteil der Haut und/oder den pH-Wert der Haut erfassen.

Zur messtechnischen Überprüfung des Vorhandenseins von auf die Hautoberfläche aufzutragender Substanzen kann die Messvorrichtung mindestens eine Sensoreinrichtung aufweisen, wobei die Sensoreinrichtung durch Erfassen eines in einer Substanz befindlichen Markers feststellt, ob sich die mit dem Marker markierte Substanz auf der zu untersuchenden Hautoberfläche befindet.

Der Marker sollte vorzugsweise keine Wechselwirkungen mit den chemischen Komponenten der Substanz ausüben.

Der Marker ist vorzugsweise nicht von der Haut resorbierbar. Der Marker kann aus Partikeln bestehen, wobei die Partikel eine derartige Größe aufweisen, dass sie nicht in die Haut eindringen können.

Der Marker kann eine fluoreszierende Substanz sein und die Sensoreinrichtung kann den Marker mit Hilfe von Fluoreszenzmessungen erfassen.

Ein Verfahren zur messtechnischen Erfassung der Veränderung von Parametern der Haut von Lebewesen auf einer zu untersuchenden Hautoberfläche in Abhängigkeit von der Einwirkzeit einer Substanz kann folgende Schritte aufweisen:
- Messen der Hautparameter auf der unbehandelten, zu untersuchenden Hautoberfläche und Speichern der gemessenen Hautparameter,
- Ausgeben einer auf die zu untersuchende Hautoberfläche aufzutragenden Substanz und Auftragen der Substanz auf die zu untersuchende Hautoberfläche,
- Identifizieren der ausgegebenen Substanz,
- Speichern des Zeitpunktes der Ausgabe der Substanz,
- erneutes Messen der Hautparameter nach einer oder mehreren vorgegebenen Einwirkzeiten und
- Speichern des Zeitpunktes der mindestens einen erneuten Messung der Hautparameter zur Erfassung der Einwirkzeit der Substanz.

Die ausgegebene Menge der Substanz kann gemessen und gespeichert werden.

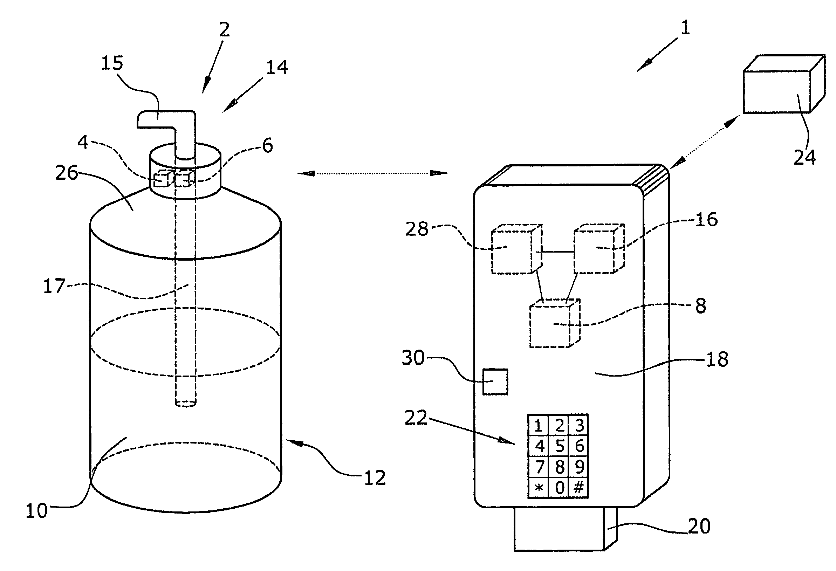

Im Folgenden wird unter Bezugnahme auf die Zeichnungen ein Ausführungsbeispiel näher erläutert. Es zeigt schematisch
- Figur 1: ein erfindungsgemäßes System zur messtechnischen Erfassung der Veränderung von Parametern der Haut von Lebewesen.

Figur 1 zeigt ein System zur messtechnischen Erfassung der Veränderung von Parametern der Haut von Lebewesen. Das System 1 besteht aus einer Spendereinrichtung 2 und einem Messgerät 18. Die Spendereinrichtung 2 weist einen Behälter 12 auf, der die Substanz 10 aufnimmt. Die Substanz 10 ist vorzugsweise eine Hautcreme. Der Behälter 12 kann mit einem Deckel 26 verschlossen werden. Der Deckel 26 weist eine Öffnung auf, durch die die Leitungsröhre 17 geführt ist, die Teil einer Dosiereinrichtung 14 ist. Am oberen Ende der Leitungsröhre 17 ist ein Dosierkopf 15 angeordnet, der einstückig mit der Leitungsröhre 17 ausgebildet ist. Der Dosierkopf 15 weist an dem freien Ende eine Öffnung auf.

Durch Betätigen des Dosierkopfes 15 der Dosiervorrichtung 14 wird die Substanz 10 aus dem Behälter 12 durch die Leitungsröhre 17, den Dosierkopf 15 und die Öffnung des Dosierkopfes nach außen auf die Haut gepumpt.

Die Spendereinrichtung 2 weist weiter eine Prozessoreinheit 4 und eine Speichereinheit 6 auf. Die Prozessoreinheit 4 weist vorzugsweise eine Uhr zum Zuordnen eines Zeitpunktes zu gemessenen Daten auf.

Bei Betätigung des Dosierkopfes 15 der Dosiereinrichtung 14 wird der Zeitpunkt der Betätigung von der Prozessoreinheit 4 erfasst und in der Speichereinheit 6 gespeichert. Ebenso wird die Substanzmenge, die nach außen gepumpt wird, erfasst und in der Speichereinheit 6 gespeichert. Der Zeitpunkt der Betätigung und die Substanzmenge werden einander zugeordnet in der Speichereinheit 6 gespeichert. In der Speichereinheit 6 wird beispielsweise gespeichert, dass zu einem Zeitpunkt a die Substanzmenge b ausgegeben worden ist.

Das Messgerät 18 weist ebenfalls eine Prozessoreinheit 28 und eine Speichereinheit 16 auf. Die Prozessoreinheit 28 weist ebenfalls eine Uhr auf. Zudem weist das Messgerät 18 eine Signalausgabe 8, eine Tastatur 22 und eine Sensoreinrichtung mit Sensoren 20 auf. Die Sensoreinrichtung kann beispielsweise Sensoren 20 zur Erfassung der Hautelastizität und/oder der Hautfarbe und/oder des Fettanteils der Haut und/oder des Talganteils der Haut und/oder des PH-Wertes der Haut und/oder die Hauttemperatur und/oder der Hautfeuchtigkeit aufweisen.

Der Zeitpunkt, an dem die Sensoreinheit die Hautparameter misst, wird von der Prozessoreinheit 28 erfasst. Ebenso werden die zugehörigen Messergebnisse, die bei einer Messung gemessen werden, von der Prozessoreinheit 28 erfasst. Die Daten werden dann an die Speichereinheit 16 weitergegeben und dort gespeichert. Der Zeitpunkt der Messung und die zugehörigen Messergebnisse werden einander zugeordnet in der Speichereinheit 16 gespeichert. In der Speichereinheit 16 wird somit gespeichert, dass zu einem Zeitpunkt x beispielsweise die Hautparameter y, z, w gemessen worden sind, wobei die Hautparameter y, z, w beispielsweise die Hautelastizität, die Hautfarbe und der pH-Wert der Haut sein können. Ebenso kann festgestellt werden, wie viel Zeit vergangen ist seit der Betätigung der Dosiereinrichtung 14 der Spendeeinrichtung 2. Da das Auftragen der Creme näherungsweise mit dem Betätigen der Dosiereinrichtung 14 gleichgesetzt werden kann, entspricht die seit der Betätigung der Dosiereinrichtung 14 vergangene Zeit der Einwirkzeit der Substanz 10 bzw. Creme.

Das Messgerät 18 kann zusätzlich eine Raumklimasensoreinrichtung 30 aufweisen. Die Prozessoreinheit 4 der Spendereinrichtung 2 und die Prozessoreinheit 28 des Messgerätes 18 können über ein lokales drahtloses Netzwerk miteinander kommunizieren. Somit können die Daten, die in der Speichereinheit 6 gespeichert sind auch an die Prozessoreinheit 28 übermittelt werden und von dort aus in die Speichereinheit 16 gespeichert werden. Alternativ können auch die Daten die in der Speichereinheit 16 gespeichert sind über die Prozessoreinheit 28 zu der Prozessoreinheit 4 gesendet werden und dort in der Spelchereinheit 6 gespeichert werden. Das heißt, es kann auch nur eine der beiden Speichereinheiten 6, 16 verwendet werden.

Vor Gebrauch des Systems 1 wird die in der Spendereinrichtung 2 befindliche Substanz 10 anhand einer der Substanz 10 zugeordneten Identifikationscodierung identifiziert. Diese Identifizierung kann beispielsweise dadurch geschehen, dass die Identifikationscodierung über die Tastatur 22 in das Messgerät 18 eingegeben wird. Diese Identifikationscodierung wird dann von der Prozessoreinheit 28 in der Speichereinheit 16 gespeichert und/oder an die Prozessoreinheit 4 gesendet und dort in der Speichereinheit 6 gespeichert. Jegliche von der Prozessoreinheit 4 und/oder von der Prozessoreinheit 28 im Folgenden erfassten Daten werden dieser Identifikationscodierung zugeordnet und in der Speichereinheit 6 und/oder in der Speichereinheit 16 gespeichert. Das heißt, die in der Speichereinheit 6 und/oder in der Speichereinheit 16 gespeicherten Daten können einer Substanz 10 zugeordnet werden.

Zusätzlich kann ein bestimmtes Zeitintervall über die Tastatur 22 eingestellt werden, das in der Speichereinheit 16 und/oder in der Speichereinheit 6 gespeichert wird. Sobald nach dem Betätigen der Dosiereinrichtung 14 das in der Speichereinheit 16 und/oder in der Speichereinheit 6 gespeicherten Zeitintervall abgelaufen ist, wird ein Signal von der Signalausgabeeinheit 8 ausgegeben. Das Signal soll den Probanden oder die Probandin daran erinnern, eine Hautmessung vorzunehmen. Es können auch mehrere Zeitintervalle eingegeben und in der Speichereinheit 16 und/oder in der Speichereinheit 6 gespeichert werden, wobei jeweils nach Ablauf der jeweiligen Zeitintervalle eine Messung mit dem Messgerät 18 vorgenommen werden soll. Auch können bestimmte Uhrzeiten in der Speichereinheit 16 und/oder in der Speichereinheit 6 gespeichert werden, nach denen ein Signal ausgegeben wird, um den Probanden bzw. die Probandin daran zu erinnern eine Messung durchzuführen oder die Substanz 10 bzw. die Creme auf die Hautoberfläche aufzutragen. Das Auftragen der Creme kann näherungsweise mit dem Betätigen der Dosiereinrichtung 14 gleichgesetzt werden.

Die Daten, die in der Speichereinheit 6 und/oder Speichereinheit 16 gespeichert sind, können an einen externen Datenempfänger gesendet werden. Das Messgerät 18 kann beispielsweise über ein USB-Kabel an einen Computer angeschlossen werden und die Daten können dann über das Internet an den externen Datenempfänger gesendet werden.

Alternativ kann sich in dem Messgerät 18 und/oder der Spendeeinrichtung 2 eine gängige Speicherkarte, wie beispielsweise eine SD-Karte befinden. Die Daten können durch die Prozessoreinheit 4 und/oder durch die Prozessoreinheit 28 auf diese Speicherkarte übertragen werden. Die Karte kann in ein Datenlesegerät eines Computers eingeführt werden und dann können die Daten ebenfalls über das Internet an einen externen Datenempfänger gesendet werden.

Zusätzlich kann sich in der Substanz 10 ein Marker befinden. Der Marker kann, nachdem die Substanz 10 auf die Hautoberfläche aufgetragen worden ist, durch einen Sensor der Sensoreinheit des Messgerätes 18 erfasst werden. Der Marker ist nicht von der Haut resorbierbar und es finden keine Wechselwirkungen zwischen dem Marker und der chemischen Komponenten der Substanz 10 statt. Der Marker kann beispielsweise aus Partikeln bestehen, die eine derartige Größe aufweisen, dass sie nicht in die Haut eindringen können. Weiter kann der Marker eine fluoreszierende Substanz sein, die von einem Sensor des Messgerätes 18 mit Hilfe von Fluoreszenzmessung erfasst werden kann.

Bei einem Verfahren zur messtechnischen Erfassung der Veränderung von Parametern der Haut von Lebewesen auf einer zu untersuchenden Hautoberfläche in Abhängigkeit von der Einwirkzeit einer Substanz werden die Hautparameter auf der unbehandelten, zu untersuchenden Hautoberfläche mit Hilfe der Sensoren 20 des Messgerätes 18 gemessen und die gemessenen Hautparameter werden in der Speichereinheit 16 und/oder in der Speichereinheit 6 gespeichert. Auch kann der Zeitpunkt der Messung gespeichert werden.

In einem weiteren Schritt wird die aufzutragende Substanz identifiziert. Dies kann beispielsweise durch Eingabe einer Identifikationscodierung über die Tastatur 22 des Messgerätes 18 geschehen.

Im nächsten Schritt wird eine Substanz, die auf die zu untersuchende Hautoberfläche aufgetragen werden soll und die in einem Behälter 12 der Spendeeinrichtung 2 angeordnet ist, mit Hilfe der Dosiereinrichtung 14 der Spendeeinrichtung 2 ausgegeben und auf die zu untersuchende Hautoberfläche aufgetragen.

Im nächsten Schritt wird der Zeitpunkt der Ausgabe der Substanz 10 gespeichert. Der Zeitpunkt der Ausgabe der Substanz 10 kann mit dem Auftragen der Substanz 10 auf die Hautoberfläche nahezu gleichgesetzt werden. Zusätzlich kann auch die Menge der ausgegebenen Substanz 10 erfasst und gespeichert werden. Der Zeitpunkt der Ausgabe der Substanz 10 und, falls die Menge der Substanz 10 erfasst wird, die ausgegebene Menge der Substanz 10 werden einander und der Identifikationscodierung zugeordnet in der Speichereinheit 16 und/oder der Speichereinheit 6 gespeichert.

In dem nächsten Schritt werden die Hautparameter nach einer oder mehreren vorgegebenen Einwirkzeiten mit Hilfe der Sensoren 20 des Messgerätes 18 erneut gemessen. Die Einwirkzeit entspricht der Zeit, die seit der Ausgabe der Substanz 10 vergangen ist.

Im nächsten Schritt wird der Zeitpunkt der mindestens einen erneuten Messung der Hautparameter und die gemessenen Hautparameter gespeichert. Der Zeitpunkt der Messung und die Messergebnisse werden einander zugeordnet und der Identifikationscodierung zugeordnet in der Speichereinheit 16 und/oder der Speichereinheit 6 gespeichert.

## Patentansprüche

1. System zur messtechnischen Erfassung der Veränderung von Parametern der Haut von Lebewesen auf einer zu untersuchenden Hautoberfläche in Abhängigkeit von der Einwirkzeit einer Substanz (10), mit
- mindestens einer Messvorrichtung (18) zum Messen der Hautparameter,
- mindestens einer Spendeeinrichtung (2), die eingerichtet ist, durch Betätigung eine dieser Spendeeinrichtung (2) zugeordnete und auf die zu untersuchende Hautoberfläche auftragbare Substanz (10) auszugeben, und
- einer Speicherheit (6,16),
wobei die Messvorrichtung (18) eingerichtet ist, den Zeitpunkt der Betätigung der Spendeeinrichtung (2) und den Zeitpunkt mindestens einer Messung der Hautparameter zur Erfassung der Einwirkzeit der Substanz (10) sowie die gemessenen Hautparameter, die dem Zeitpunkt der Messung zugeordnet sind, in der Speichereinheit (6,16) aufzuzeichnen
**dadurch gekennzeichnet, dass**
die Messvorrichtung (18) mit der mindestens einen Spendeeinrichtung (2) über ein lokales drahtloses Netzwerk gekoppelt ist.

2. System nach Anspruch 1, **dadurch gekennzeichnet, dass** die Spendeeinrichtung (2) eingerichtet ist, die Substanz in einer vorgegebenen Menge auszugeben.

3. System nach einem der Ansprüche 1 bis 2, **dadurch gekennzeichnet, dass** die Messvorrichtung (18) eingerichtet ist, nach einem einstellbaren Zeitintervall oder mehreren einstellbaren Zeitintervallen jeweils ein Erinnerungssignal auszugeben.

4. System nach Anspruch 1, **dadurch gekennzeichnet, dass** die Messvorrichtung eingerichtet ist, die in der Speichereinheit (6,16) gespeicherten Daten an einen externen Datenempfänger zu senden.

5. System nach einem der Ansprüche 1 bis 4, **dadurch gekennzeichnet, dass** die Messvorrichtung (18) eine Raumklima-Sensoreinrichtung (30) aufweist.

6. System nach einem der Ansprüche 1 bis 5, **dadurch gekennzeichnet, dass** die Messvorrichtung (18) eingerichtet ist, die Hautelastizität und/oder die Hautfarbe und/oder den Fettanteil der Haut und/oder den Talganteil der Haut und/oder die Hautfeuchte und/oder den pH-Wert der Haut zu erfassen.

7. System nach einem der Ansprüche 1 bis 6, **dadurch gekennzeichnet, dass** zur messtechnischen Überprüfung des Vorhandenseins von auf die Hautoberfläche aufzutragender Substanzen (10) die Messvorrichtung (18) mindestens eine Sensoreinrichtung aufweist, wobei die Sensoreinrichtung eingerichtet ist, durch Erfassen eines in einer Substanz (10) befindlichen Markers festzustellen, ob sich die mit dem Marker markierte Substanz auf der zu untersuchenden Hautoberfläche befindet.

8. System nach Anspruch 7, **dadurch gekennzeichnet, dass** das System eine mit einem Marker markierte Substanz (10) aufweist, wobei der Marker von der Haut nicht resorbierbar ist.

9. System nach Anspruch 8, **dadurch gekennzeichnet, dass** der Marker aus Partikeln besteht, wobei die Partikel eine derartige Größe aufweisen, dass sie nicht in die Haut eindringen können.

10. System nach einem der Ansprüche 7 bis 9, **dadurch gekennzeichnet, dass** der Marker eine fluoreszierende Substanz ist und die Sensoreinrichtung eingerichtet ist, den Marker mit Hilfe von Fluoreszenzmessungen zu erfassen.

## Claims

1. A system for measuring changes in skin parameters of living beings on a skin surface to be examined as a function of the residence time of a substance (10), comprising
- at least one measuring apparatus (18) for measuring the skin parameters,
- at least one dispenser means (2) configured to dispense, when operated, a substance (10) associated with this dispenser means (2) and applicable on the skin surface to be examined, and
- a memory means (6, 16),
the measuring apparatus (18) being configured to record in said memory device (6, 16) the time of actuation of the dispenser means (2) and the time of the at least one measurement of the skin parameters for the detection of the residence time of the substance (10), as well as the skin parameters measured which are associated with the time of measurement,
wherein
the measuring apparatus (18) is coupled to the at least one dispenser means (2) via a local wireless network.

2. The system of claim 1, wherein the dispenser means (2) is configured to dispense the substance in a predefined quantity.

3. The system of one of claims 1 to 2, wherein the measuring apparatus (18) is configured to issue a reminder signal respectively after a settable time interval or a plurality of settable time intervals.

4. The system of claim 1, wherein the measuring apparatus is configured to transmit the data stored in the storage unit (6, 16) to at least one external data receiver.

5. The system of one of claims 1 to 4, wherein the measuring apparatus (18) comprises a room climate sensor means (30).

6. The system of one of claims 1 to 5, wherein the measuring apparatus (18) is configured to detect the skin elasticity and/or the color of the skin and/or the fat level of the skin and/or the sebum level of the skin and/or the skin humidity and/or the pH value of the skin.

7. The system of one of claims 1 to 6, wherein the measuring apparatus (18) comprises at least one sensor means with which the presence of substances (10) to be applied on the skin surface can be checked by measurement techniques, the sensor means being configured to detect a marker present in a substance (10) in order to determine whether the substance (10) marked with the marker is present on the skin surface to be examined.

8. The system of claim 7, wherein the system comprises a substance (10) marked with said marker, the marker not being resorbable by the skin.

9. The system of claim 8, wherein the marker consists of particles, the particles being of such a size that they cannot penetrate into the skin.

10. The system of one of claims 7 to 9, wherein the marker is a fluorescent substance and the sensor means is configured to detect the marker by means of fluorescence measurement.

## Revendications

1. Système de détection par métrologie de la variation de paramètres de la peau d'êtres vivants sur une surface de peau à examiner en fonction du temps d'action d'une substance (10), avec
- au moins un dispositif de mesure (18) pour la mesure des paramètres de peau,
- au moins un dispositif de distribution (2) qui est aménagé pour délivrer, par actionnement, une substance (10) affectée à ce dispositif de distribution (2) et pouvant être appliquée sur la surface de peau à examiner, et
- une unité de mémoire (6, 16),
le dispositif de mesure (18) étant aménagé pour enregistrer dans l'unité de mémoire (6, 16) le moment de l'actionnement du dispositif de distribution (2) et le moment d'au moins une mesure des paramètres de peau pour la détection de la durée d'action de la substance (10) ainsi que les paramètres de peau mesurés qui sont affectés au moment de la mesure
**caractérisé en ce que**
le dispositif de mesure (18) est couplé au dispositif de distribution (2) au moins au nombre de un par le biais d'un réseau sans fil local.

2. Système selon la revendication 1, **caractérisé en ce que** le dispositif de distribution (2) est aménagé pour délivrer la substance à raison d'une quantité prédéfinie.

3. Système selon l'une des revendications 1 à 2, **caractérisé en ce que** le dispositif de mesure (18) est aménagé pour délivrer respectivement un signal de rappel après un intervalle de temps réglable ou plusieurs intervalles de temps réglables.

4. Système selon la revendication 1, **caractérisé en ce que** le dispositif de mesure est aménagé pour envoyer à un récepteur de données externe les données enregistrées dans l'unité de mémoire (6, 16).

5. Système selon l'une des revendications 1 à 4, **caractérisé en ce que** le dispositif de mesure (18) présente un dispositif de capteur (30) de climat ambiant.

6. Système selon l'une des revendications 1 à 5, **caractérisé en ce que** le dispositif de mesure (18) est aménagé pour détecter l'élasticité de la peau et/ou la couleur de la peau et/ou la proportion de graisse de la peau et/ou la proportion de sébum de la peau et/ou l'humidité de la peau et/ou le pH de la peau.

7. Système selon l'une des revendications 1 à 6, **caractérisé en ce que**, pour le contrôle par métrologie de la présence de substances (10) à appliquer sur la surface de la peau, le dispositif de mesure (18) présente au moins un dispositif de capteur, le dispositif de capteur étant aménagé pour constater, par la détection d'un marqueur situé dans une substance (10), si la substance marquée par le marqueur se trouve sur la surface de la peau à examiner.

8. Système selon la revendication 7, **caractérisé en ce que** le système présente une substance (10) marquée par un marqueur, le marqueur n'étant pas résorbable par la peau.

9. Système selon la revendication 8, **caractérisé en ce que** le marqueur se compose de particules, les particules présentant une dimension telle qu'elles ne peuvent pas pénétrer dans la peau.

10. Système selon l'une des revendications 7 à 9, **caractérisé en ce que** le marqueur est une substance fluorescente et **en ce que** dispositif de capteur est aménagé pour détecter le marqueur à l'aide de mesures de la fluorescence.
